(19) Europäisches Patentamt
European Patent Office
Office européen des brevets

(11) EP 0 736 536 B1

(12) # FASCICULE DE BREVET EUROPEEN

(45) Date de publication et mention de la délivrance du brevet:
**20.06.2001 Bulletin 2001/25**

(51) Int Cl.[7]: **C07F 3/02**, C08F 10/00, C07C 2/30, C07C 51/15, C07C 29/48

(21) Numéro de dépôt: **96400731.4**

(22) Date de dépôt: **04.04.1996**

(54) **Dialkylmagnésium à longues chaînes, son procédé de préparation et ses applications**

Langkettige Dialkylmagnesiumverbindungen, deren Herstellung und Verwendung

Long chain dialkyl magnesium compounds, their preparation and use

(84) Etats contractants désignés:
**AT BE CH DE DK ES FI GB GR IE IT LI LU MC NL PT SE**
Etats d'extension désignés:
**LT LV SI**

(30) Priorité: **07.04.1995 FR 9504203**

(43) Date de publication de la demande:
**09.10.1996 Bulletin 1996/41**

(73) Titulaires:
- **ENICHEM S.p.A.**
  **20124 Milano (IT)**
- **UNIVERSITE DES SCIENCES ET TECHNOLOGIES DE LILLE**
  **59655 Villeneuve d'Ascq (FR)**

(72) Inventeurs:
- **Pelletier, Jean-François**
  **83130 La Garde (FR)**
- **Bujadoux, Karel**
  **59240 Dunkerque (FR)**
- **Olonde, Xavier**
  **59960 Neuville en Ferrain (FR)**
- **Adisson, Emmanuel**
  **62510 Arques (FR)**
- **Mortreux, André**
  **59510 Hem (FR)**
- **Chenal, Thomas**
  **59650 Villeneuve d'Ascq (FR)**

(74) Mandataire: **Chaillot, Geneviève**
**Cabinet CHAILLOT,**
**16-20, avenue de L'Agent Sarre,**
**B.P. 74**
**92703 Colombes Cédex (FR)**

(56) Documents cités:
**US-A- 3 670 038**

- **CHEMISCHE BERICHTE, vol. 126, 1993, pages 1371-1383, XP002006062 BOGDANOVIC, B. ET AL.: "DIORGANOMAGNESIUM COMPOUNDS FROM MAGNESIUM, HYDROGEN, AND 1-ALKENES AND THEIR APPLICATION IN SYNTHESIS"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention porte sur un dialkylmagnésium à longues chaînes, sur un procédé permettant de le préparer, ainsi que sur ses applications.

Il est bien connu que la réaction d'insertions multiples de l'éthylène ou des a-oléfines dans une liaison Métal-Carbone se réalise facilement et rapidement avec les métaux de transition des Groupes III à VIII de la Classification Périodique. Les applications qui en découlent, telles que la polymérisation et l'oligomérisation, sont largement développées de nos jours au stade industriel. Les produits ainsi fabriqués sont des α-oléfines supérieures ou des chaînes hydrocarbonées linéaires ou branchées de très haute masse moléculaire. Cependant, la réactivité chimique de ces produits se trouve fortement amoindrie par le fait que les extrémités de ces chaînes hydrocarbonées ne sont plus liées au métal ayant servi à leur croissance.

Parmi les autres métaux, le cas de l'aluminium est intéressant. La réaction des trialkylaluminiums (par exemple d'$AlEt_3$) avec l'éthylène, découverte par K. Ziegler, est plus connue sous le nom d'"Aufbaureaktion". Cette dernière s'effectue en général sous des pressions moyennes d'éthylène, à des températures supérieures à 115°C. Les conditions de réaction définissent la nature des produits fabriqués :

- la synthèse d'oléfines terminales emploie des températures auxquelles une réaction de β hydrogène élimination est fréquente. La croissance des chaînes et leur terminaison s'effectuent à des vitesses comparables. Typiquement, la réaction s'effectue à 200 - 250°C, sous des pressions d'éthylène de 130-150 bars. Les produits majoritaires sont des a-oléfines linéaires comprenant 4 à 8 atomes de carbone ;
- la formation de trialkylaluminiums lourds s'opère à 115-130°C et à 135 bars (brevet américain US-A-3 450 735). Les produits de réaction, de type AlRR'R", possèdent des longueurs de chaîne avec une répartition statistique allant de 6 à 14 carbones. L'application principale consiste à oxyder ces trialkylaluminiums lourds pour produire des alcools gras utilisés dans l'industrie des détergents.

Un autre procédé, récemment mis au point, pour la synthèse de trialkylaluminiums lourds, est décrit dans la demande de brevet européen EP-A-0 574 854. La réaction d'oligomérisation de l'éthylène est assurée de façon catalytique par des complexes métallocéniques à base de métaux du groupe des actinides ($Cp^*_2UCl_2$ ; Cp* = pentaméthylcyclopentadiényle), activés par un aluminoxane ou un agent non-coordinant de la classe des perfluoroborates. Les chaînes en croissance sont transférées sur l'aluminium qui est présent sous forme d'$AlR_3$. La distribution des alkylaluminiums lourds ainsi produits suit en général une distribution de Poisson. Leurs longueurs de chaînes varient de 4 à 22 carbones.

La réaction s'effectue sous des conditions de température et de pression comprises entre respectivement 0 - 100°C et 1 - 70 bars, pendant 10 à 40 minutes. Les avantages cités dans ce mode opératoire sont l'utilisation de conditions plus douces et l'obtention de produits de plus grande pureté, car on diminue notablement la formation de polyéthylène en tant que sous-produit formé par réaction de β hydrogène élimination.

En ce qui concerne le magnésium, les dialkyl-magnésiums sont habituellement préparés par dismutation ou par alkylation des complexes de Grignard.

La réaction d'oligomérisation de l'éthylène avec des dialkylmagnésiums (l'analogue de l'"Aufbaureaktion" décrite ci-dessus), est décrite par D.B. Malpass, dans J. Organomet. Chem., Library (1980), 9, 1, et par H.G. Richey, dans Inorganic Reactions and Methods (Ed. : A.P. Hogen), VCH, New York, (1989), vol. 10, section 5.4.2.5.1. Elle permet de produire un mélange statistique de dialkylmagnésiums avec des longueurs de chaînes comprises entre 4 et 20 carbones. Les conditions de réaction sont de 120°C, 150 bars d'éthylène pendant 12 heures. De plus, la présence d'une base forte, telle que la quinuclidine, en quantité stoechiométrique est nécessaire. Des sous-produits (α-oléfines) sont générés simultanément et représentent environ 15% des composés. De plus, la base forte se trouve par la suite mélangée (ou complexée) aux produits de réaction et nécessite des étapes de purification coûteuses. De plus, la longueur des chaînes alkyle est limitée par les réactions de terminaison (β hydrogène élimination) ; la formation de dialkyl-magnésiums lourds exigerait l'utilisation de plus hautes pressions.

B. Bogdanovic et al. [Chem. Ber., (1993), 126, 1371-1383] ont optimisé dernièrement l'hydromagnésation, qui consiste à synthétiser des dialkylmagnésiums à partir de $MgH_2$ et d'une α-oléfine en présence d'un catalyseur ($MtCl_4$, Mt représentant un métal du Groupe IVB de la Classification Périodique). La réaction s'effectue en deux étapes :

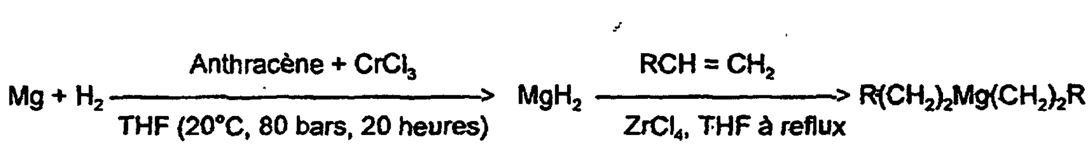

Malgré une étape complexe pour la synthèse du $MgH_2$ intermédiaire, les avantages cités par B. Bogdanovic et al. se définissent en termes de meilleurs rendements et de procédé plus économique. Cependant, les longueurs des chaînes alkyle liées au magnésium sont définies par la taille de l'α-oléfine utilisée lors de la synthèse. Ces longueurs ne vont pas au-delà de 20 atomes de carbone.

US-A-3 670 038 décrit un procédé de fabrication d'alkylmagnésiums supérieurs à partir d'alkylmagnésiums inférieurs, par réaction de l'éthylène, du propylène ou du butène-1 et d'un dialkylmagnésium dissous dans un solvant de réaction non complexant, en l'absence de catalyseur.

Les présents inventeurs se sont attachés aux dialkylmagnésiums, qui sont de par leur nature plus aptes que les alkylaluminiums correspondants à réaliser des réactions de "valorisation". Ils ont découvert qu'il est possible de polymériser l'éthylène à l'aide d'un complexe particulier à base de lanthanide comme catalyseur, puis de transférer les chaînes en croissance sur le magnésium d'un dialkylmagnésium intervenant en tant qu'agent de transfert de grande efficacité. De plus, ils ont découvert que les conditions de température et de pression de cette réaction sont plus favorables que celles préconisées par D.B. Malpass et H.G. Richey, ainsi que par B. Bogdanovic et al. (supra), avec des temps de réaction nettement diminués. Par ailleurs, les caractéristiques des chaînes liées au magnésium (masse moléculaire et polydispersité) sont facilement contrôlées par les conditions de synthèse. Le mélange des produits de réaction qui est obtenu est caractérisé par une distribution statistique très étroite qui est favorable aux diverses applications envisagées.

La présente invention a donc d'abord pour objet un dialkylmagnésium à longues chaînes, représenté par la formule (I) suivante :

$$R - (CH_2 - CH_2)_n - Mg - (CH_2\text{-}CH_2)_{n'} - R', (E\text{-}O\text{-}E')_x \qquad (I)$$

dans laquelle :

- R et R' représentent chacun indépendamment un reste hydrocarboné en $C_1$-$C_{20}$, ramifié ou non, substitué ou non, de type alkyle, cycloalkyle ou aralkyle ;
- n et n', identiques ou sensiblement identiques, représentent un nombre moyen d'enchaînements $-CH_2-CH_2-$ tel que le nombre moyen d'atomes de carbone de chacune des deux chaînes portées par Mg soit supérieur à 20, en particulier soit supérieur à 40 ;
- E et E', identiques ou différents, représentent chacun un radical alkyle, linéaire ou cyclique, ramifié ou non ; et
- $0 \le x \le 2$, x étant la valeur moyenne du nombre de moles d'éther E-O-E' complexé au dialkylmagnésium.

A titre d'exemples de restes alkyle entrant dans la définition de R et R', on peut citer les restes méthyle, éthyle, n-propyle, n-butyle, s-butyle, t-butyle et n-hexyle.

Le nombre moyen d'atomes de carbone de chacune des deux chaînes portées par Mg est, comme indiqué, généralement supérieur à 20, ce nombre pouvant aller jusqu'à 300, voire même jusqu'à 500.

E-O-E' représente un éther complexé au dialkylmagnésium proprement dit.

A titre d'exemples de tels éthers, on peut citer le diéthyl éther, le méthyl isobutyl éther, le di-n-butyl éther et le di-isoamyl éther.

De façon générale, on peut citer les radicaux alkyle en $C_2$-$C_5$ comme exemples illustrant E et E'.

Ces dialkylmagnésiums de formule (I) ont avantageusement un indice de polydispersité $\overline{Mw}/\overline{Mn}$ compris entre 1,1 et 2, et encore mieux, entre 1,1 et 1,5.

La présente invention a également pour objet un procédé de préparation d'un dialkylmagnésium représenté par la formule (I) suivante :

$$R - (CH_2 - CH_2)_n - Mg - (CH_2\text{-}CH_2)_{n'} - R', (E\text{-}O\text{-}E')_x \qquad (I)$$

dans laquelle :

- R et R' représentent chacun indépendamment un reste hydrocarboné en $C_1$-$C_{20}$, ramifié ou non, substitué ou non, de type alkyle, cycloalkyle ou aralkyle ;
- n et n', identiques ou sensiblement identiques, représentent un nombre moyen d'enchaînements $-CH_2-CH_2-$ tel que le nombre moyen d'atomes de carbone de chacune des deux chaînes portées par Mg soit supérieur à 2 ;
- E et E', identiques ou différents, représentent chacun un radical alkyle, linéaire ou cyclique, ramifié ou non ; et
- $0 \le x \le 2$, x étant la valeur moyenne du nombre de moles d'éther E-O-E' complexé au dialkylmagnésium.

caractérisé par le fait que, dans un solvant hydrocarboné, anhydre et désoxygéné, on polymérise de l'éthylène à l'aide d'un catalyseur représenté par la formule (II) :

$$Cp^*_2MCl_2Li(OEt_2)_2 \qquad (II)$$

dans laquelle :

- Cp* représente un ligand pentaméthylcyclopentadiényle ;
- M est un métal choisi parmi le scandium, l'yttrium et les métaux de la série des lanthanides ; et
- Et représente éthyle,

en présence d'un dialkylmagnésium, soluble dans ledit solvant, représenté par la formule (III) :

$$R - Mg - R', (E\text{-}O\text{-}E')_x \qquad (III)$$

dans laquelle R, R', E, E' et x sont tels que définis ci-dessus, en tant qu'agent de transfert de chaînes, et qu'on arrête la réaction avant que la polydispersité du produit polymère obtenu ne dépasse 2, de préférence 1,5.

Comme dialkylmagnésium de formule (III), on peut citer, entre autres, le n-butyléthylmagnésium. On peut également citer le di-n-hexylmagnésium ou encore un dialkylmagnésium soluble répondant à la formule (I) qui vient d'être définie et préparé par le procédé qui vient d'être décrit, les branches R-$(CH_2\text{-}CH_2)_n$- et R'-$(CH_2\text{-}CH_2)_{n'}$- du dialkylmagnésium (I) étant alors les branches R- et R'- du dialkylmagnésium (III).

L'écriture R - Mg - R', $(E\text{-}O\text{-}E')_x$ avec x > 0 signifie qu'un éther E-O-E' a été complexé au dialkylmagnésium proprement dit. Dans le cas où le dialkylmagnésium serait insoluble dans le milieu réactionnel, l'addition d'éther permet de le solubiliser sous la forme (III) avec x > 0. Dans ce cas, l'éther reste complexé au produit final de formule (I) avec x > 0.

Comme métal M du catalyseur (II), on peut citer le samarium, le néodyme et l'yttrium.

Le rapport molaire Mg/M est avantageusement compris entre 5 et 10000, de préférence entre 30 et 1000.

Comme solvant hydrocarboné pour la réaction, on utilise notamment un hydrocarbure aliphatique linéaire ou cyclique, saturé ou insaturé, tel que l'heptane, le cyclohexane, la décaline, l'isodécane ou un mélange d'alcanes lourds en $C_9$-$C_{12}$, tel que l'ISOPAR® L, ou un hydrocarbure aromatique, substitué ou non, tel que le toluène, le xylène ou le mésitylène.

La réaction selon l'invention est conduite dans des conditions douces de pression et de température, lesquelles varient avantageusement respectivement de 1 à 10 bars, notamment de 1 à 2 bars, et de 20 à 120°C, notamment de 40 à 80°C. La durée de la réaction dépend du rapport Mg/M ; elle est généralement comprise entre 2 minutes et 100 heures, notamment entre 1 et 3 heures.

De façon générale, il est souhaitable d'arrêter la réaction avant ou lors de l'apparition d'un précipité correspondant à une augmentation franche de la consommation d'éthylène.

Comme on le montrera ultérieurement, le premier stade des produits de la réaction (avant l'apparition d'un précipité) correspond à la réaction de formation des dialkylmagnésiums de formule (I), tandis que le second stade correspond à la production de polyéthylène. Dans l'optique de produire des dialkylmagnésiums exempts de polyéthylène, il est donc nécessaire d'arrêter la réaction avant l'apparition du deuxième stade.

La longueur des chaînes $(CH_2\text{-}CH_2)_n$- ou -$(CH_2\text{-}CH_2)_{n'}$- est fonction des conditions opératoires, plus particulièrement du rapport Mg/M et du temps de réaction.

La présente invention porte également sur un procédé de polymérisation d'au moins un monomère polaire sur le dialkylmagnésium de formule (I), tel que défini ci-dessus, n et n' étant tels que le nombre moyen d'atomes de carbone de chacune des deux chaînes portées par Mg soit supérieur à 20, en tant que macroinitiateur.

Les monomères polaires sont notamment choisis parmi les monomères (méth)acryliques, comme les méthacrylates d'alkyle (de méthyle, d'éthyle, d'isopropyle, d'isobutyle, etc.) ; le (méth)acrylonitrile ; les vinylpyridines comme la vinyl-4 pyridine ; et les lactones, comme l'ε-caprolactone.

On conduit généralement la polymérisation du (ou des) monomère(s) polaire(s) en solution dans un solvant hydrocarboné, tel que le toluène, à une température de -78°C à 100°C, pendant 1 à 100 heures ; on arrête la polymérisation par hydrolyse par l'éthanol ou l'eau, et on récupère un copolymère biséquencé éthylène - comonomère(s) polaire(s) par filtration et/ou extraction à l'aide d'un solvant solubilisant l'homopolymère du comonomère polaire correspondant. On peut citer l'acétone à titre d'exemple.

La présente invention porte également sur diverses applications des dialkylmagnésiums de l'invention.

Ainsi, ces dialkylmagnésiums peuvent être appliqués en tant que macroinitiateurs pour la polymérisation de monomères polaires, tels que ceux définis ci-dessus. On obtient alors des copolymères biséquencés ayant une séquence de polyéthylène qui sont utiles notamment comme compatibilisants pour les mélanges des deux homopolymères correspondants.

Ces dialkylmagnésiums peuvent également servir à la fabrication d'alcools primaires linéaires gras, utiles dans l'industrie des détergents biodégradables, par oxydation et hydrolyse ; à la production d'α-oléfines par décomposition thermique ; à la production d'acides par réaction avec $CO_2$ ; et pour fixer un radical de type $R\text{-}(CH_2\text{-}CH_2)_n\text{-}$ sur une molécule organique ou un principe actif, afin d'en accroître la liposolubilité, ce qui présente un intérêt dans l'industrie pharmaceutique.

Les exemples suivants illustrent l'invention sans toutefois en limiter la portée.

- Les composés (I) ont été notés P-Mg-P' ou même $MgP_2$ pour simplifier. BEM désigne le n-butyléthylmagnésium.

- Les dialkylmagnésiums P - Mg - P' synthétisés, pour lesquels au moins l'un parmi P et P' comprend un nombre d'atomes de carbone supérieur à 30, ont été caractérisés par chromatographie par perméation de gel (GPC) après précipitation dans l'alcool éthylique pour hydrolyse, puis récupération par filtration, suivant le processus indiqué ci-dessous (Analyse A).

  Un prélèvement de 10 $cm^3$ du milieu réactionnel est introduit dans 200 $cm^3$ d'alcool éthylique. Les produits issus de l'hydrolyse, conformément au schéma réactionnel :

$$P\text{-}Mg\text{-}P' + 2ROH \rightarrow PH + P'H + Mg(OR)_2$$

précipitent et sont récupérés par filtration, lavés avec 20 $cm^3$ d'alcool éthylique, puis séchés à 80°C durant 24 heures.

. Les dialkylmagnésiums P - Mg - P' synthétisés, pour lesquels P et P' comprennent chacun un nombre moyen d'atomes de carbone compris entre 2 et 30, ont été caractérisés par chromatographie en phase gaz (GC) après hydrolyse à basse température sous atmosphère inerte suivant le processus indiqué ci-dessous (Analyse B).

On prélève du milieu réactionnel un échantillon de 1 $cm^3$ que l'on dilue dans 10 $cm^3$ de pentane désoxygéné. La solution obtenue est refroidie à -20°C, puis on ajoute, sous agitation, goutte à goutte, 5 $cm^3$ d'eau désoxygénée, et on laisse réagir pendant 1 heure. La réaction est conforme au schéma réactionnel :

$$P\text{-}Mg\text{-}P' + 2H_2O \xrightarrow{-20^\circ C} PH + P'H + Mg(OH)_2$$

La phase organique est ensuite récupérée par décantation, séchée et soumise à l'analyse chromatographique en phase gazeuse.

- Les dialkylmagnésiums synthétisés ont également été identifiés par réaction avec $CO_2$ selon la réaction :

$$P - Mg - P' + 2CO_2 \xrightarrow{2H_3O^+} PCO_2H + P'CO_2H + Mg^{2+}$$

pour former des acides, lesquels peuvent être caractérisés par spectroscopie de résonance magnétique nucléaire, spectrométrie de masse et infrarouge.

- Par ailleurs, des réactions d'alkylation de $SnCl_4$ par les dialkylmagnésiums ont été effectuées suivant la réaction ci-dessous :

$$2\ P - Mg - P' + Sncl_4 \rightarrow P_2\text{-}Sn\text{-}P'_2 + 2\ Mgcl_2.$$

La réaction a été conduite à 80°C pendant 2 heures dans l'ISOPAR L comme solvant. Le produit résultant ($P_2$-Sn-$P'_2$) a été analysé par GPC (Analyse C).

- Les dialkylmagnésiums peuvent aussi être identifiés par spectroscopie infrarouge des produits dérivés obtenus par thermolyse. Effectivement, portés à une température élevée (supérieure à 125°C), les dialkylmagnésiums se décomposent en α-oléfines et dihydrure de magnésium par réaction de β hydrogène élimination. Ainsi, l'élévation du taux d'insaturation pendant la thermolyse peut être observée.

- Ces dialkylmagnésiums sont, dans tous les cas, caractérisés par une masse moléculaire moyenne en nombre ($\overline{Mn}$), une masse moléculaire moyenne en poids ($\overline{Mw}$), ainsi qu'un indice de polydispersité ($\overline{Mw}/\overline{Mn}$). On indique également la valeur Mp qui désigne la masse moléculaire au sommet de la distribution. Le nombre moyen d'atomes de carbone dans chacune des deux chaînes liées au magnésium a été calculé à partir des $\overline{Mn}$.

Exemple 1 :

Sous atmosphère inerte, 64,4 mg (0,1 mmole) de $Cp^*_2SmCl_2Li(OEt_2)_2$ sont mis en solution dans 20 $cm^3$ de toluène. Une quantité de n-butyléthylmagnésium est ajoutée à cette solution pour obtenir un rapport molaire Mg/Sm = 10, et le tout est agité durant 2 heures à température ambiante (20°C).

Un ballon à double enveloppe, équipé d'une agitation, est séché à 120°C, puis purgé avec de l'argon. On y introduit 500 $cm^3$ d'ISOPAR®L anhydre que l'on porte à 80°C. Le solvant est ensuite saturé avec l'éthylène sous 1,2 bar. La solution préparée précédemment est alors injectée dans le réacteur. La consommation d'éthylène est alors enregistrée par des rotamètres. La réaction est conduite pendant 5 minutes. Le rendement en dialkylmagnésium à longues chaînes P-Mg-P' est de 4,4 g (soit 4,3 g d'éthylène consommés). L'éthylène est ensuite évacué et remplacé par de l'argon. Un échantillon est prélevé, puis soumis à l'Analyse A telle qu'indiquée ci-dessus. Les masses moléculaires $\overline{Mn}$, $\overline{Mw}$ et Mp sont respectivement les suivantes : 1900, 2400, 2390. La longueur moyenne de chaque chaîne liée au magnésium vaut alors 1900 / 14 = 136 carbones (cf Tableau 1).

Exemple 2

On reproduit le mode opératoire de l'Exemple 1 en utilisant une quantité de $Cp^*_2SmCl_2Li(OEt_2)_2$ de 0,2 mmole et un rapport Mg/Sm de 50, et en conduisant la polymérisation pendant 54 minutes.

Au bout de ces 54 minutes, le mélange réactionnel est refroidi légèrement, et le produit réactionnel est récupéré par filtration sur verre fritté. On obtient ainsi un solide blanchâtre, dont un échantillon est soumis à l'Analyse A.

Les conditions opératoires et les résultats de l'Analyse A sont également rapportés dans le Tableau 1.

Exemples 3 à 13

On reproduit le mode opératoire de l'Exemple 1, en faisant varier le rapport molaire Mg/Sm et/ou le temps de réaction, comme indiqué dans le Tableau 1, et en utilisant une quantité de $Cp^*_2SmCl_2Li(OEt_2)_2$ de 0,05 mmole dans le cas de l'Exemple 11.

Les conditions opératoires et les résultats des Analyses A sont rapportés également dans le Tableau 1 ci-après.

TABLEAU 1

| Exemples | Rapport molaire Mg/Sm | Temps de réaction (minutes) | Rendement en g d'éthylène consommés | $\overline{Mn}$ | $\overline{Mw}$ | Mp | $\overline{Mw}/\overline{Mn}$ | Nombre moyen d'atomes de C de chaque chaîne liée au magnésium |
|---|---|---|---|---|---|---|---|---|
| 1 | 10 | 5 | 4,3 | 1900 | 2400 | 2390 | 1,3 | 136 |
| 2 | 50 | 54 | 30 | 1290 | 1620 | 1370 | 1,3 | 92 |
| 3 | 10 | 5 | 4,7 | 1870 | 2190 | 2190 | 1,2 | 134 |
| 4 | 20 | 5 | 3,3 | 690 | 900 | 800 | 1,3 | 49 |
| 5 | 50 | 5 | 2,9 | 400 | 530 | 490 | 1,3 | 29 |
| 6 | 1000 | 1480 | 93 | 460 | 500 | 560 | 1,1 | 33 |
| 7 | 10 | 1 | 0,8 | 540 | 690 | 560 | 1,3 | 39 |
| 8 | 10 | 2,5 | 2,3 | 970 | 1300 | 1240 | 1,3 | 69 |
| 9 | 10 | 7 | 5,8 | 1640 | 2320 | 2420 | 1,4 | 117 |
| 10 | 10 | 2,5 | 2,23 | 1070 | 1400 | 1400 | 1,3 | 76 |
| 11 | 20 | 5,5 | 2,25 | 980 | 1280 | 1170 | 1,3 | 70 |
| 12 | 100 | 75,3 | 38 | 1050 | 1590 | 1050 | 1,5 | 75 |
| 13 | 10 | 10 | 10,1 | 2120 | 3370 | 3070 | 1,6 | 151 |

La comparaison des résultats respectivement des Exemples 1 et 3 et des Exemples 8 et 10 permet de vérifier la bonne reproductibilité du procédé selon l'invention.

La comparaison des résultats des Exemples 3, 7, 8 et 9 permet de noter que les masses moléculaires au pic (Mp) augmentent régulièrement en fonction du temps, tout en conservant une polydispersité étroite. On peut ainsi émettre l'hypothèse que toutes les chaînes croissent de façon similaire au cours du temps.

La comparaison des résultats des Exemples 10 et 11, traduisant des essais effectués à isoconversion, montre que les masses moléculaires des produits fabriqués dépendent directement de la quantité de n-butyléthylmagnésium introduite au départ et non pas de la quantité de samarium.

On peut remarquer (Exemple 13) qu'à des valeurs plus élevées de la polydispersité ($\overline{Mw}/\overline{Mn}$), le rendement augmente (par comparaison avec l'Exemple 1), ce qui signifie l'apparition d'un second produit caractérisé comme étant du polyéthylène. L'obtention de ces valeurs plus élevées de la polydispersité correspond au passage au second stade tel que défini ci-dessus.

Exemple 14

Dans cet Exemple, on a étudié de façon détaillée l'évolution de la pureté des produits en fonction du stade de la réaction.

On reproduit l'Exemple 1, excepté que l'on utilise un rapport molaire Mg/Sm de 50. On poursuit la réaction jusqu'à son terme et l'on effectue des prélèvements d'un volume de 20 $cm^3$ chacun tout au long de la réaction. Les produits issus de ces prélèvements ont été soumis à l'Analyse A. Les résultats sont indiqués dans le Tableau 2.

TABLEAU 2

| Prélèvement | Temps (minutes) | Débit d'éthylène (l/h) | $\overline{Mn}$ | $\overline{Mw}$ | Mp | $\overline{Mw}/\overline{Mn}$ | Nombre moyen d'atomes de C dans chaque chaîne liée au magnésium | Rendement en g d'éthylène consommés |
|---|---|---|---|---|---|---|---|---|
| A | 1,6 | 42 | 485 | 590 | 450 | 1,2 | 35 | 1,10 |
| B | 8,3 | 27 | 580 | 700 | 600 | 1,2 | 41 | 5,50 |
| C | 18,3 | 22 | 900 | 1110 | 1070 | 1,2 | 64 | 10,15 |
| D | 20,3 | 41 | 950 | 1200 | 1120 | 1,3 | 68 | 11,05 |
| E | 22,1 | 91 | 1130 | 1710 | 1240 | 1,5 | 81 | 13,50 |
| F | 27,9 | 79 | 1420 | 2620 | 1350 | 1,8 | Précipitation | 23,40 |
| G | 30 | 52 | 1550 | 3030 | 1410 | 2,0 | " | 26,35 |
| H | 35 | 15 | 1720 | 6470 | 1470 | 3,8 | " | 29 |
| I | 41 | 6 | 1530 | 6080 | 1410 | 4,0 | " | 30,10 |
| J | 55 | 0 | 1860 | 10960 | 1480 | 5,9 | " | 30,70 |

L'ensemble des prélèvements permet de distinguer les deux stades de la réaction. En effet, le premier stade comprend les prélèvements A à E (synthèse de P-Mg-P' proprement dit), alors que le second stade englobe les prélèvements F à J.

Il est confirmé qu'au cours de la même réaction, les masses moléculaires moyennes en nombre et en poids, ainsi que la masse moléculaire au pic, augmentent régulièrement, et que la polydispersité reste étroite durant le premier stade. A compter du prélèvement F, une précipitation a lieu et la polydispersité augmente de manière importante. L'allure des courbes GPC montre que la distribution devient bimodale. Le dialkylmagnésium ayant précipité, la réaction continue en produisant du polyéthylène de plus haute masse moléculaire. L'étude de l'évolution des spécifications des dialkylmagnésiums en fonction du temps montre bien que la pureté de ces derniers est fonction du stade d'arrêt de la réaction. La valeur maximale de la masse moléculaire au pic de la distribution principale correspondant aux dialkylmagnésiums P-Mg-P' exempts de polyéthylène atteint alors 1240 g/mole. De ce fait, il est possible ici de synthétiser des chaînes alkyle P et P' liées au magnésium pouvant comporter chacune en moyenne 81 atomes de carbone.

Exemple 15 : Synthèse de $MgP_2$ par le système $Cp^*_2NdCl_2Li(OEt_2)_2$ + BEM à 80°C

On reproduit le mode opératoire de l'Exemple 14 en remplaçant le samarium par le néodyme.

Les conditions opératoires et les résultats sont présentés dans le Tableau 3.

TABLEAU 3

| Prélèvement | Temps (minutes) | Débit d'éthylène (l/h) | $\overline{Mn}$ | $\overline{Mw}$ | Mp | $\overline{Mw}/\overline{Mn}$ | Nombre moyen d'atomes de C dans chaque chaîne liée au magnésium | Rendement en g d'éthylène consommés |
|---|---|---|---|---|---|---|---|---|
| A | 5 | 31 | 700 | 880 | 670 | 1,2 | 50 | 2,80 |
| B | 16,6 | 15 | 870 | 1090 | 970 | 1,2 | 62 | 7,70 |
| C | 33,9 | 13 | 1200 | 1500 | 1410 | 1,3 | 86 | 12,45 |
| D | 43,1 | 14 | 1390 | 1750 | 1700 | 1,3 | 99 | 14,70 |
| E | 45,4 | 69 | 1560 | 2080 | 1860 | 1,3 | 111 | 15,85 |
| F | 46,3 | 90 | 1745 | 2675 | 1840 | 1,5 | 125 | 17,40 |
| G | 48,6 | 61 | 2070 | 3600 | 2000 | 1,7 | Précipitation | 20,40 |
| H | 52,1 | 55 | 2540 | 4580 | 2360 | 1,8 | " | 24,40 |
| I | 96 | 4 | 4400 | 10680 | 7630 | 2,4 | " | 44,10 |

Exemple 16 : Synthèse de $MgP_2$ par le système $Cp^*_2YCl_2Li(OEt_2)_2$ + BEM

On reproduit le mode opératoire de l'Exemple 14 en remplaçant le samarium par l'yttrium avec Mg/Y = 50.

Un seul prélèvement a été effectué à 23 minutes. La quantité d'éthylène consommée est de 13,5 g. Un échantillon a alors été soumis à l'Analyse A. Les résultats sont :

$\overline{Mn}$ = 1080
$\overline{Mw}$ = 1650
Mp = 1360
$\overline{Mw}$ / $\overline{Mn}$ = 1,5
Nombre moyen d'atomes de carbone dans chaque chaîne liée à Mg = 97.

Exemple 17 : Synthèse de $MgP_2$ par le système $Cp^*_2SmCl_2Li(OEt_2)_2$ + BEM à 40°C

On reproduit le mode opératoire de l'Exemple 14 en opérant à 40°C.

Les résultats de l'Analyse A conduite sur le prélèvement effectué après 8 minutes de polymérisation sont les suivants :

$\overline{Mn}$ = 740
$\overline{Mw}$ = 890
Mp = 700
$\overline{Mw}$ / $\overline{Mn}$ = 1,2
Nombre moyen d'atomes de carbone dans chaque chaîne liée à Mg = 53

Exemple 18 : Synthèse de $MgP_2$ par le système $Cp^*_2SmCl_2Li(OEt_2)_2$ + BEM à 60°C

On reproduit le mode opératoire de l'Exemple 14 en opérant à 60°C.

Les conditions opératoires et les résultats sont présentés dans le Tableau 4.

TABLEAU 4

| Prélèvement | Temps (minutes) | Débit d'éthylène (l/h) | $\overline{Mn}$ | $\overline{Mw}$ | Mp | $\overline{Mw}/\overline{Mn}$ | Nombre moyen d'atomes de C dans chaque chaîne liée au magnésium | Rendement en g d'éthylène consommés |
|---|---|---|---|---|---|---|---|---|
| A | 5 | 20 | 760 | 900 | 720 | 1,2 | 54 | 1,90 |
| B | 16,6 | 12 | 910 | 1160 | 910 | 1,3 | 65 | 6,10 |
| C | 21,3 | 9 | 990 | 1280 | 1025 | 1,3 | 71 | 7,35 |
| D | 28,7 | 7,5 | 980 | 1270 | 1110 | 1,3 | 70 | 8,70 |
| E | 31,5 | 21 | 1050 | 1440 | 1120 | 1,4 | 75 | 9,40 |
| F | 34,4 | 17 | 1200 | 4630 | 1200 | 3,8 | Précipitation | 10,75 |
| G | 41,7 | 9 | 1400 | 11280 | 1200 | 8,1 | " | 12,70 |

Exemple 19 : Synthèse de $MaP_2$ à partir du système $Cp^*_2SmCl_2Li(OEt_2)_2$ + BEM à 80°C

On reproduit le mode opératoire de l'Exemple 14, en utilisant le xylène comme solvant.

L'Analyse A conduite sur le prélèvement effectué après 1 h 48 min de polymérisation a donné les résultats suivants :

$\overline{Mn}$ = 3070
$\overline{Mw}$ = 4530
Mp = 4900
$\overline{Mw}$ / $\overline{Mn}$ = 1,5
Nombre moyen d'atomes de carbone dans chaque chaîne liée à Mg = 219

Exemple 20 : Synthèse d'un $MgP_2$ léger à partir du système $Cp^*_2SmCl_2Li(OEt_2)_2$ + BEM

On reproduit l'Exemple 1, excepté que l'on utilise un rapport molaire Mg/Sm de 100 et qu'on remplace le toluène par l'heptane. La durée de la polymérisation est de 5 minutes.

Le produit de réaction, isolé par évaporation à sec de l'heptane, a été soumis à l'Analyse B après l'hydrolyse conduite dans les conditions indiquées. Les résultats sont indiqués dans le Tableau 5 ci-après.

Exemple 21 : Synthèse d'un $MaP_2$ léger à partir du système $Cp^*_2SmCl_2Li\ (OEt_2)_2$ + di-n-hexylmagnésium

On reproduit l'Exemple 20, excepté que l'on utilise un rapport Mg/Sm de 134 et qu'on remplace le n-butyléthylmagnésium par le di-n-hexylmagnésium. La durée de la polymérisation est de 64,3 minutes.

Le produit de réaction, isolé par évaporation à sec de l'heptane, a été soumis à l'Analyse B, après l'hydrolyse conduite dans les conditions indiquées.

Les résultats sont également indiqués dans le Tableau 5 ci-après.

TABLEAU 5

| Alcanes linéaires issus de l'hydrolyse de P-Mg-P' Nombre de carbones | Exemple 20 | Exemple 21 |
|---|---|---|
| | % molaire | % molaire |
| 6 | 24,7 | 3,5 |
| 8 | 26,8 | 7,7 |
| 10 | 22,5 | 14,8 |
| 12 | 12,1 | 23,0 |
| 14 | 6,5 | 14,6 |
| 16 | 4,1 | 11,2 |
| 18 | 1,8 | 8,0 |
| 20 | 0,8 | 5,6 |
| 22 | 0,4 | 4,0 |
| 24 | 0,1 | 2,8 |
| 26 | 0,1 | 2,0 |
| 28 | 0 | 1,5 |
| 30 | 0 | 1,2 |
| 32 | 0 | 0,9 |
| 34 | 0 | 0,7 |

La répartition des fractions molaires des alcanes suit une loi de Poisson, qui est une distribution statistique décrite par :

$$Xp = \frac{e^{-\lambda} . \lambda^{X}}{X!}$$

où Xp est la fraction molaire avec x insertions d'éthylène et λ le coefficient de la distribution de Poisson, égal au nombre moyen d'éthylène additionné par liaison Mg-C. La valeur de λ est 2,35 et 3,54 pour les dialkylmagnésiums obtenus

dans les Exemples respectivement 20 et 21. Il est connu que la loi de Poisson est caractéristique d'une distribution très étroite, ce qui confirme les résultats obtenus par les présents inventeurs.

On peut par ailleurs constater que, dans le cas de l'Exemple 21, la distribution de Poisson est très fidèlement respectée, car l'organomagnésien de départ est symétrique.

Exemple 22

On reproduit l'Exemple 1, excepté que le rapport Mg/Sm = 50, que le solvant est l'heptane et que l'on conduit la réaction durant 30 minutes. Le solvant est alors évaporé à siccité, et 8,6 g de produit sont isolés.

Un échantillon n° 1 est soumis à l'analyse A après hydrolyse par l'alcool éthylique.

Un échantillon n° 2 est soumis à une thermolyse à 200°C durant 3 heures, puis à l'analyse A après hydrolyse par l'alcool éthylique.

Les échantillons n° 1 et n° 2 sont ensuite analysés par spectroscopie infrarouge pour le dosage des doubles liaisons de type vinyle, vinylidène et trans-vinylène.

Les résultats de l'analyse A et du dosage infrarouge des fonctions insaturées sont présentés dans le Tableau 6 :

TABLEAU 6

| Ech. n° | $\overline{Mn}$ | $\overline{Mw}$ | $\overline{Mw}/\overline{Mn}$ | Double liaison pour 1000C | | | |
|---|---|---|---|---|---|---|---|
| | | | | Vinyle | Vinylidène | Trans-vinylène | Total |
| 1 | 696 | 798 | 1,1 | 0,325 | 0,577 | <0,005 | 0,902 |
| 2 | 761 | 921 | 1,2 | 2,518 | 0,479 | 4,445 | 7,442 |

Exemple 23

On reproduit l'Exemple 1, excepté que l'on utilise un rapport molaire Mg/Sm de 50 et que le temps de réaction est de 25 minutes.

Les résultats des Analyses A et C telles que définies ci-dessus sont présentés dans le Tableau 7.

TABLEAU 7

| Analyse | $\overline{Mn}$ | $\overline{Mw}$ | Mp | $\overline{Mw}/\overline{Mn}$ |
|---|---|---|---|---|
| A | 1025 | 1240 | 1220 | 1,2 |
| C | 2360 | 3580 | 4100 | 1,5 |

L'Analyse C révèle une distribution bimodale qui s'explique ici par le fait que la réaction entre $MgP_2$ et $SnCl_4$ a été incomplète. Cependant, on peut remarquer que la masse moléculaire au pic (Mp) se trouve augmentée d'un facteur 3,5. Ces résultats prouvent que les chaînes issues de la multiple insertion de l'éthylène sont effectivement liées au magnésium.

Exemple 24 : Préparation d'un copolymère éthylène - méthacrylate de méthyle

On reproduit les conditions de l'Exemple 1, excepté que l'on utilise un rapport Mg/Sm de 50.

Lorsqu'on arrive à 5 minutes de polymérisation, on effectue un prélèvement de 20 $cm^3$ du mélange réactionnel, que l'on soumet à l'Analyse A, dont les résultats sont les suivants :

$\overline{Mn}$ = 400
$\overline{Mw}$ = 525
Mp = 485
$\overline{Mw}/\overline{Mn}$ = 1,3
Nombre moyen d'atomes de carbone dans chaque chaîne liée à Mg = 29

puis on injecte 50 mmoles de méthacrylate de méthyle.

Au bout de 5 minutes, le produit intermédiaire organométallique est hydrolysé à l'éthanol à la même température (80°C), ce qui stoppe la réaction. Ensuite le copolymère est précipité par dilution dans l'heptane. On récupère par filtration 3,2 g du copolymère attendu.

L'Analyse A conduite sur ce copolymère a donné les résultats suivants :

$\overline{Mn}$ = 720
$\overline{Mw}$ = 990
Mp = 920
$\overline{Mw}/\overline{Mn}$ = 1,4

Exemple 25 : Préparation d'un copolymère éthylène - méthacrylate de méthyle

On reproduit les conditions de l'Exemple 1, excepté que l'on utilise un rapport Mg/Sm de 50.
Lorsqu'on arrive à 30 minutes de polymérisation, on effectue un prélèvement de 20 $cm^3$ du mélange réactionnel, que l'on soumet à l'analyse A, dont les résultats sont les suivants.

$\overline{Mn}$ = 640
$\overline{Mw}$ = 880
Mp = 710
$\overline{Mw}/\overline{Mn}$ = 1,4
Nombre moyen d'atomes de carbone dans chaque chaîne liée à Mg = 45
Quantité d'éthylène consommée = 12,4 g

Le milieu réactionnel est alors ramené de 80°C à -78°C, et on y injecte alors 150 mmoles de méthacrylate de méthyle. Au bout de 2 heures, le produit intermédiaire organométallique est hydrolysé à l'éthanol à la même température (-78°C), ce qui stoppe la réaction. Ensuite le copolymère est précipité par dilution dans l'heptane à la température ambiante. On récupère par filtration 16,7 g du copolymère attendu.
Un échantillon du produit brut ainsi obtenu (15,5 g) est extrait à 56°C durant 4 jours dans un appareil d'extraction Kumagawa de 500 $cm^3$, avec l'acétone comme solvant. On récupère, dans la cartouche, 8,2 g de produit que l'on soumet à l'analyse A, laquelle donne les résultats suivants :

$\overline{Mn}$ = 890
$\overline{Mw}$ = 1000
Mp = 920
$\overline{Mw}/\overline{Mn}$ = 1,1

Exemple 26 : Préparation d'un copolymère éthylène - méthacrylate de méthyle

On utilise le dialkylmagnésien à longues chaînes préparé dans l'Exemple 2, pour lequel les résultats de l'analyse A sont rapportés dans le Tableau 1.
2 mmoles de ce P-Mg-P' sont solubilisées dans 100 $cm^3$ de toluène anhydre et désoxygéné. La solution est ensuite refroidie à -30°C. 100 mmoles de méthacrylate de méthyle sont alors injectés. La réaction est conduite durant 48 h. Le polymère est précipité dans le pentane, puis filtré. Un échantillon du produit brut ainsi obtenu (7,7 g) est extrait à 56°C pendant 4 jours dans un appareil d'extraction Kumagawa de 500 $cm^3$, avec l'acétone comme solvant. On récupère, dans la cartouche, 7,2 g de produit que l'on soumet à l'analyse A, laquelle donne les résultats suivants :

$\overline{Mn}$ = 1480
$\overline{Mw}$ = 1940
Mp = 1580
$\overline{Mw}/\overline{Mn}$ = 1,3

Exemple 27 : Préparation d'un copolymère éthylène - ε-caprolactone

On reproduit les conditions de l'Exemple 1, excepté que l'on utilise un rapport Mg/Sm de 50.
Lorsqu'on arrive à 22 minutes de polymérisation, on effectue un prélèvement de 20 $cm^3$ du mélange réactionnel, que l'on soumet à l'Analyse A, laquelle donne les résultats suivants :

$\overline{Mn}$ = 1100
$\overline{Mw}$ = 1325
Mp = 1250
$\overline{Mw}/\overline{Mn}$ = 1,2

Nombre moyen d'atomes de carbone dans chaque chaîne liée à Mg = 79

et, à 24 minutes, on injecte 50 mmoles d'ε-caprolactone.

Au bout d'une heure, le produit intermédiaire organométallique est hydrolysé à l'éthanol à la même température (80°C), ce qui stoppe la réaction.

Un échantillon du produit brut ainsi obtenu (8,6 g) est extrait à 56°C pendant 10 jours dans un appareil d'extraction Kumagawa de 500 $cm^3$, avec de l'acétone comme solvant. On récupère, dans la cartouche, 1 g de produit que l'on soumet à l'Analyse A, laquelle donne les résultats suivants :

$\overline{Mn}$ = 1760
$\overline{Mw}$ = 2230
Mp = 2230
$\overline{Mw}/\overline{Mn}$ = 1,26

La fraction solubilisée présente une distribution bimodale avec un pic correspondant au copolymère éthylène - ε-caprolactone, et un second pic correspondant à l'homopolymère d'ε-caprolactone.

Exemple 28 : Application à la synthèse d'acides carboxyliques saturés

On reproduit l'Exemple 1, excepté que l'on utilise un rapport molaire Mg/Sm de 100, et qu'on remplace l'ISOPAR® L par le toluène. La polymérisation est conduite pendant 5 minutes.

Une fois la polymérisation terminée, on élimine l'éthylène par dégazage, puis on refroidit la solution à 15°C sous argon. Ensuite, du $CO_2$ sec est mis à buller directement dans la solution durant 20 minutes. Puis on réalise trois extractions en milieu aqueux acide ($H_2O$/HCl 5%). La phase organique est recueillie et séchée sur $Na_2SO_4$ pendant 48 heures sous agitation.

Après concentration de la solution, le produit brut est analysé par spectrométrie de masse ($FAB^+$). On a pu identifier les ions moléculaires de m/e = 101 à 297, correspondant aux acides $CH_3-(CH_2)_3-CO_2H$ à $CH_3-(CH_2)_{17}-CO_2H$ espacés chacun d'un motif $-CH_2-CH_2-$ (d'une masse de 28 g).

Exemple 29

On reproduit l'Exemple 1, excepté que 0,74 g d'éther diéthylique (correspondant à $Et_2O$/Mg = 2) ont été ajoutés à 0,55 g de n-butyléthylmagnésium en solution dans 20 cm3 de toluène. Après une heure, la solution obtenue est ajoutée à 64,4 mg (0,1 mmole) de $Cp^*_2SmCl_2Li(OEt_2)_2$ (Mg/Sm = 50).

La polymérisation est conduite durant 1015 secondes avec un débit d'éthylène constant et égal à 40 l/h. On a obtenu 13,65 g de dialkylmagnésium à longues chaînes.

## Revendications

**1.** Procédé de préparation d'un dialkylmagnésium représenté par la formule (I) suivante :

$$R - (CH_2 - CH_2)_n - Mg - (CH_2 - CH_2)_{n'} - R', (E\text{-}O\text{-}E')_x \qquad (I)$$

dans laquelle :

- R et R' représentent chacun indépendamment un reste hydrocarboné en $C_1$-$C_{20}$, ramifié ou non, substitué ou non, de type alkyle, cycloalkyle ou aralkyle ;
- n et n', identiques ou sensiblement identiques, représentent un nombre moyen d'enchaînements $-CH_2-CH_2-$ tel que le nombre moyen d'atomes de carbone de chacune des deux chaînes portées par Mg soit supérieur à 2 ;
- E et E', identiques ou différents, représentent chacun un radical alkyle, linéaire ou cyclique, ramifié ou non ; et
- $0 \leq x \leq 2$, x étant la valeur moyenne du nombre de moles d'éther E-O-E' complexé au dialkylmagnésium,

caractérisé par le fait que, dans un solvant hydrocarboné, anhydre et désoxygéné, on polymérise de l'éthylène à l'aide d'un catalyseur représenté par la formule (II) :

$$Cp^*_2MCl_2Li(OEt_2)_2 \quad (II)$$

dans laquelle :

- $Cp^*$ représente un ligand pentaméthylcyclopentadiényle ;
- M est un métal choisi parmi le scandium, l'yttrium et les métaux de la série des lanthanides ; et
- Et représente éthyle,

en présence d'un dialkylmagnésium, soluble dans ledit solvant, représenté par la formule (III) :

$$R - Mg - R', (E\text{-}O\text{-}E')_x \quad (III)$$

dans laquelle R, R', E, E' et x sont tels que définis ci-dessus, en tant qu'agent de transfert de chaînes, et qu'on arrête la réaction avant que la polydispersité du produit polymère obtenu ne dépasse 2, de préférence 1,5.

**2.** Procédé selon la revendication 1, caractérisé par le fait qu'on utilise comme dialkylmagnésium de formule (III) le n-butyléthylmagnésium ou le di-n-hexylmagnésium ou un dialkylmagnésium de la formule (I) telle que définie à la revendication 1 ou préparé par le procédé tel que défini à la revendication 1.

**3.** Procédé selon l'une des revendications 1 et 2, caractérisé par le fait qu'on utilise un catalyseur dans lequel M représente le samarium, le néodyme ou l'yttrium.

**4.** Procédé selon l'une des revendications 1 à 3, caractérisé par le fait que le rapport molaire Mg/M est compris entre 5 et 10000, de préférence entre 30 et 1 000.

**5.** Procédé selon l'une des revendications 1 à 4, caractérisé par le fait qu'on utilise, comme solvant, un hydrocarbure aliphatique linéaire ou cyclique, saturé ou insaturé, tel que l'heptane, le cyclohexane, la décaline, l'isodécane ou un mélange d'alcanes lourds en $C_9$-$C_{12}$, ou un hydrocarbure aromatique, substitué ou non, tel que le toluène, le xylène ou le mésitylène.

**6.** Procédé selon l'une des revendications 1 à 5, caractérisé par le fait que l'on conduit la réaction sous une pression de 1 à 10 bars, de préférence de 1 à 2 bars.

**7.** Procédé selon l'une des revendications 1 à 6, caractérisé par le fait que l'on conduit la réaction à une température de 20 à 120°C, de préférence de 40 à 80°C.

**8.** Procédé selon l'une des revendications 1 à 7, caractérisé par le fait que l'on conduit la réaction pendant une durée de 2 minutes à 100 heures, notamment de 1 à 3 heures.

**9.** Procédé selon l'une des revendications 1 à 8, caractérisé par le fait que l'on arrête la réaction avant ou lors de l'apparition d'un précipité correspondant à une augmentation franche de la consommation d'éthylène.

**10.** Dialkylmagnésium à longues chaînes, représenté par la formule (I) suivante :

$$R - (CH_2 - CH_2)_n - Mg - (CH_2 - CH_2)_{n'} - R', (E\text{-}O\text{-}E')_x \quad (I)$$

dans laquelle :

- R et R' représentent chacun indépendamment un reste hydrocarboné en $C_1$-$C_{20}$, ramifié ou non, substitué ou non, de type alkyle, cycloalkyle ou aralkyle ;
- n et n', identiques ou sensiblement identiques, représentent un nombre moyen d'enchaînements $-CH_2-CH_2-$ tel que le nombre moyen d'atomes de carbone de chacune des deux chaînes portées par Mg soit supérieur à 20 ;
- E et E', identiques ou différents, représentent chacun un radical alkyle, linéaire ou cyclique, ramifié ou non ; et

- $0 \leq x \leq 2$, x étant la valeur moyenne du nombre de moles d'éther E-O-E' complexé au dialkylmagnésium.

**11.** Dialkylmagnésium selon la revendication 10, caractérisé par le fait que le nombre moyen d'atomes de carbone de chacune des deux chaînes portées par Mg est supérieur à 40.

**12.** Dialkylmagnésium selon l'une des revendications 10 et 11, caractérisé par un indice de polydispersité compris entre 1,1 et 2, notamment entre 1,1 et 1,5.

**13.** Procédé de polymérisation d'au moins un monomère polaire sur le dialkylmagnésium de formule (I) conforme à l'une des revendications 10 à 12, en tant que macroinitiateur.

**14.** Procédé selon la revendication 13, caractérisé par le fait que l'on utilise au moins un monomère polaire choisi parmi les (méth)acrylates d'alkyle, le (méth)acrylonitrile, les vinylpyridines et les lactones.

**15.** Procédé selon l'une des revendications 13 et 14, caractérisé par le fait que l'on conduit la polymérisation du (ou des) monomère(s) polaire(s) en solution dans un solvant hydrocarboné, à une température de -78°C à 100°C, pendant 1 à 100 heures, qu'on arrête la polymérisation par hydrolyse par l'éthanol ou l'eau, et qu'on récupère un copolymère biséquencé éthylène - comonomère(s) polaire(s) par filtration ou extraction à l'aide d'un solvant solubilisant l'homopolymère du comonomère polaire correspondant.

**16.** Application des dialkylmagnésiums à longues chaînes tels que définis à l'une des revendications 10 à 12, en tant que macroinitiateurs pour la polymérisation de monomères polaires, conduisant à des copolymères biséquencés ayant une séquence de polyéthylène.

**17.** Application des dialkylmagnésiums à longues chaînes tels que définis à l'une des revendications 10 à 12, à la fabrication d'alcools primaires linéaires gras, par oxydation et hydrolyse.

**18.** Application des dialkylmagnésiums à longues chaînes tels que définis à l'une des. revendications 10 à 12, à la production d'$\alpha$-oléfines par décomposition thermique.

**19.** Application des dialkylmagnésiums à longues chaînes tels que définis à l'une des revendications 10 à 12, à la production d'acides par réaction avec $CO_2$.

**20.** Application des dialkylmagnésiums à longues chaînes tels que définis à l'une des revendications 10 à 12, pour fixer un radical de type $R\text{-}(CH_2\text{-}CH_2)_n\text{-}$ où R et n sont tels que définis à la revendication 1 sur une molécule organique ou un principe actif, afin d'en accroître la liposolubilité.

## Claims

**1.** A process for the preparation of a dialkylmagnesium represented by the following formula (I) :

$$R - (CH_2 - CH_2)_n - Mg - (CH_2 - CH_2)_{n'} - R', (E\text{-}O\text{-}E')_x \qquad (I)$$

wherein :

- R and R' each independently represent a $C_1$-$C_{20}$ hydrocarbon residue, branched or not, substituted or not, of the alkyl, cycloalkyl or aralkyl type ;
- n and n', identical or substantially identical, represent an average number of $-CH_2\text{-}CH_2-$ linkages such that the average number of carbon atoms in each of the two chains borne by Mg is greater than 2 ;
- E and E', identical of different, each represent an alkyl radical, linear or cyclical, branched or not ; and
- $0 \leq x \leq 2$, x being the average value of the number of moles of E-O-E' ether complexed to the dialkylmagnesium,

characterised by the fact that ethylene is polymerised in an anhydrous de-oxygenated hydrocarbonaceous solvent by means of a catalyst represented by formula (II) :

$$Cp^*{}_2MCl_2Li(OEt_2)_2 \qquad (II)$$

in which :

- $Cp^*$ represents a pentamethylcyclopentadienyl ligand ;
- M is a metal chosen from scandium, yttrium and metals of the lanthanide series ; and
- Et represents ethyl,

in the presence of a dialkylmagnesium, soluble in the said solvent, represented by formula (III) :

$$R - Mg - R', (E\text{-}O\text{-}E')_x \qquad (III)$$

in which R, R', E, E' and x are as defined above, as a chain-transfer agent ;
and by the fact that the reaction is stopped before the polydispersity of the polymer product obtained exceeds 2, preferably 1.5.

**2.** A process in accordance with Claim 1, characterised by the fact that n-butylethylmagnesium or di-n-hexylmagnesium or a dialkylmagnesium of the formula (I) as defined in Claim 1 or prepared by the process as defined in Claim 1 is used as the dialkylmagnesium of formula (III).

**3.** A process in accordance with one of Claims 1 and 2, characterised by the fact that a catalyst is used in which M represents samarium, neodymium or yttrium.

**4.** A process in accordance with one of Claims 1 to 3, characterised by the fact that the Mg/Sm molar ratio is between 5 and 10000, preferably between 30 and 1000.

**5.** A process in accordance with one of Claims 1 to 4, characterised by the fact that the solvent is an aliphatic hydrocarbon, linear or cyclical, saturated or unsaturated, such as heptane, cyclohexane, decaline or isodecane ; or a mixture of $C_9$-$C_{12}$ heavy alkanes, or an aromatic hydrocarbon, substituted or not, such as toluene, xylene or mesitylene.

**6.** A process in accordance with one of Claims 1 to 5, characterised by the fact that the reaction is carried out at a pressure of between 1 to 10 bar, preferably between 1 and 2 bar.

**7.** A process in accordance with one of Claims 1 to 6, characterised by the fact that the reaction is carried out a a temperature between 20 and 120°C, preferably between 40 and 80°C.

**8.** A process in accordance with one of Claims 1 to 7, characterised by the fact that the reaction is carried out for a period between 2 minutes and 100 hours, particularly between 1 and 3 hours.

**9.** A process in accordance with one of Claims 1 to 8, characterised by the fact that the reaction is stopped prior to or at the point when a precipitate corresponding to a clear increase in the ethylene consumption appears.

**10.** Long-chain dialkylmagnesium represented by the following formula (I) :

$$R - (CH_2 - CH_2)_n - Mg - (CH_2 - CH_2)_{n'} - R', (E\text{-}O\text{-}E')_x \qquad (I)$$

wherein :

- R and R' each independently represent a $C_1$-$C_{20}$ hydrocarbon residue, branched or not, substituted or not, of the alkyl, cycloalkyl or aralkyl type ;
- n and n', identical or substantially identical, represent an average number of $-CH_2-CH_2-$ linkages such that the average number of carbon atoms in each of the two chains borne by Mg is greater than 20 ;
- E and E', identical or different, each represent an alkyl radical, linear or cyclical, branched or not ; and

- $0 \leq x \leq 2$, x being the average value of the number of moles of E-O-E' ether complexed to the dialkylmagnesium.

**11.** Dialkylmagnesium in accordance with Claim 10, characterised by the fact that the average number of carbon atoms in each of the two chains borne by Mg is greater than 40.

**12.** Dialkylmagnesium in accordance with one of Claims 10 and 11, characterised by a polydispersity index between 1.1 and 2, particularly between 1.1 and 1.5.

**13.** A process for the polymerisation of at least one polar monomer on the dialkylmagnesium of formula (I) as defined in one of Claims 10 to 12 as the macroinitiator.

**14.** A process in accordance with Claim 13, characterised by the fact that at least one polar monomer is used, chosen from alkyl (meth) acrylates, (meth)acrylonitrile, vinyl pyridines and lactones.

**15.** A process in accordance with one of Claims 13 and 14, characterised by the fact that the polymerisation of the polar monomer(s) is carried out in solution in a hydrocarbonaceous solvent, at a temperature between -78°C and 100°C, for a period of between 1 and 100 hours, that the polymerisation is stopped by hydrolysis with ethanol or water, and that a binary copolymer of ethylene and one or more polar comonomers is recovered by filtration and/or extraction by means of a solvent which dissolves the homopolymer of the corresponding polar comonomer.

**16.** An application of long-chain dialkylmagnesiums as defined in one of Claims 10 to 12 as macroinitiators for the polymerisation of polar monomers leading to diblock copolymers with a polyethylene block.

**17.** An application of long-chain dialkylmagnesiums as defined in one of Claims 10 to 12 in the manufacture of primary linear fatty alcohols by oxidation and hydrolysis.

**18.** An application of long-chain dialkylmagnesiums as defined in one of Claims 10 to 12 in the production of a-olefines by thermal decomposition.

**19.** An application of long-chain dialkylmagnesiums as defined in one of Claims 10 to 12 in the production of acids by reaction with $CO_2$.

**20.** An application of long-chain dialkylmagnesiums as defined in one of Claims 10 to 12 to fix a radical of the type R-$(CH_2\text{-}CH_2)_n$-, where R and n are as defined in Claim 1, on an organic molecule or an active component in order to increase their liposolubility.

**Patentansprüche**

**1.** Verfahren zur Herstellung eines Dialkylmagnesiums, dargestellt durch die folgende Formel (I):

$$R - (CH_2 - CH_2)_n - Mg - (CH_2 - CH_2)_{n'} - R', (E\text{-}O\text{-}E')_x \quad (I)$$

worin:

- R und R' jeweils unabhängig voneinander für einen verzweigten oder nicht verzweigten, substituierten oder nicht substituierten $C_1$-$C_{20}$-Kohlenwasserstoffrest vom Alkyl-, Cycloalkyl- oder Aralkyltyp stehen;
- n und n', die identisch oder im wesentlichen identisch sind, für eine mittlere Anzahl von $-CH_2\text{-}CH_2-$ Verkettungen stehen, so daß die mittlere Anzahl von Kohlenstoffatomen einer jeden der zwei vom Mg getragenen Ketten größer als 2 ist;
- E und E', die identisch oder voneinander verschieden sind, jeweils für einen geradkettigen oder cyclischen, verzweigten oder nicht verzweigten Alkylrest stehen; und
  $0 \leq x \leq 2$, wobei x der Mittelwert der Anzahl von Molen E-O-E'-Ether ist, der mit Dialkylmagnesium komplexiert ist,

dadurch gekennzeichnet, daß in einem von Wasser und Sauerstoff freien Kohlenwasserstoff-Lösungsmittel Ethylen mit Hilfe eines Katalysators polymerisiert wird, der durch die Formel (II) dargestellt ist:

$$Cp^*_2MCl_2Li(OEt_2)_2 \qquad (II)$$

worin:

- $Cp^*$ für einen Pentamethylcyclopentadienyl-Liganden steht;
- M ein unter Scandium, Yttrium und den Metallen der Lanthanoidenserie ausgewähltes Metall ist; und
- Et für Ethyl steht,

in Gegenwart eines Dialkylmagnesiums, das in dem genannten Lösungsmittel löslich und durch die Formel (III) dargestellt ist:

$$R - Mg - R', (E\text{-}O\text{-}E')_x \qquad (III)$$

worin R, R', E, E' und x wie obenstehend definiert sind,
als Kettenübertragungsreagens,
sowie dadurch, daß die Reaktion angehalten wird, bevor die Polydispersität des erhaltenen Polymerproduktes 2, vorzugsweise 1,5 übersteigt.

**2.** Verfahren nach Anspruch 1, dadurch gekennzeichnet, daß als Dialkylmagnesium der Formel (III) n-Butylethylmagnesium oder Di-n-hexylmagnesium oder ein Dialkylmagnesium der Formel (I) verwendet wird, so wie es in Anspruch 1 definiert ist, bzw. das durch das in Anspruch 1 definierte Verfahren hergestellt wurde.

**3.** Verfahren nach einem der Ansprüche 1 und 2, dadurch gekennzeichnet, daß ein Katalysator verwendet wird, bei dem M für Samarium, Neodymium oder Yttrium steht.

**4.** Verfahren nach einem der Ansprüche 1 bis 3, dadurch gekennzeichnet, daß das Molverhältnis Mg/M zwischen 5 und 10000, vorzugsweise zwischen 30 und 1000 liegt.

**5.** Verfahren nach einem der Ansprüche 1 bis 4, dadurch gekennzeichnet, daß als Lösungsmittel ein geradkettiger oder cyclischer, gesättigter oder ungesättigter aliphatischer Kohlenwasserstoff wie Heptan, Cyclohexan, Decalin, Isodecan oder eine Mischung von hochsiedenden Alkanen mit $C_9$-$C_{12}$ oder ein substituierter oder nicht substituierter aromatischer Kohlenwasserstoff wie Toluol, Xylol oder Mesitylen ist.

**6.** Verfahren nach einem der Ansprüche 1 bis 5, dadurch gekennzeichnet, daß die Umsetzung unter einem Druck von 1 bis 100 bar, vorzugsweise 1 bis 2 bar durchgeführt wird.

**7.** Verfahren nach einem der Ansprüche 1 bis 6, dadurch gekennzeichnet, daß die Umsetzung bei einer Temperatur von 20 bis 120°C, vorzugsweise 40 bis 80°C durchgeführt wird.

**8.** Verfahren nach einem der Ansprüche 1 bis 7, dadurch gekennzeichnet, daß die Umsetzung während einer Dauer von 2 min bis 100 h, insbesondere von 1 bis 3 h durchgeführt wird.

**9.** Verfahren nach einem der Ansprüche 1 bis 8, dadurch gekennzeichnet, daß die Reaktion vor dem. bzw. beim Auftreten eines Präzipitats angehalten wird, welches einer eindeutigen Erhöhung des Ethylenverbrauchs entspricht.

**10.** Dialkylmagnesium mit langen Ketten, dargestellt durch die folgende Formel (I):

$$R - (CH_2 - CH_2)_n - Mg - (CH_2 - CH_2)_{n'} -R', (E\text{-}O\text{-}E')_x \qquad (I)$$

worin:

- R und R' jeweils unabhängig voneinander für einen verzweigten oder nicht verzweigten, substituierten oder nicht substituierten $C_1$-$C_{20}$-Kohlenwasserstoffrest vom Alkyl-, Cycloalkyl- oder Aralkyltyp stehen;
- n und n', die identisch oder im wesentlichen identisch sind, für eine mittlere Anzahl von $-CH_2-CH_2-$Verkettungen stehen, so daß die mittlere Anzahl von Kohlenstoffatomen einer jeden der zwei vom Mg getragenen Ketten größer als 20 ist;
- E und E', die identisch oder voneinander verschieden sind, jeweils für einen geradkettigen oder cyclischen, verzweigten oder nicht verzweigten Alkylrest stehen; und
- $0 \leq x \leq 2$, wobei x der Mittelwert der Anzahl von Molen von E-O-E'-Ether ist, der mit Dialkylmagnesium komplexiert ist.

**11.** Dialkylmagnesium nach Anspruch 10, dadurch gekennzeichnet, daß die mittlere Anzahl von Kohlenstoffatomen einer jeden der zwei vom Mg getragenen Ketten größer als 40 ist.

**12.** Dialkylmagnesium nach einem der Ansprüche 10 und 11, gekennzeichnet durch einen Polydispersitätsindex, der zwischen 1,1 und 2, insbesondere zwischen 1,1 und 1,5 liegt.

**13.** Verfahren zum Polymerisieren von mindestens einem polaren Monomer am Dialkylmagnesium der Formel (I) nach einem der Ansprüche 10 bis 12 als Makroinitiator.

**14.** Verfahren nach Anspruch 13, dadurch gekennzeichnet, daß mindestens ein polares Monomer verwendet wird, das unter den Alkyl(meth)acrylaten, (Meth)acrylnitril, den Vinylpyridinen und den Lactonen ausgewählt ist.

**15.** Verfahren nach einem der Ansprüche 13 und 14, dadurch gekennzeichnet, daß die Polymerisierung des polaren Monomers bzw. der polaren Monomere in Lösung in einem Kohlenwasserstoff-Lösungsmittel bei einer Temperatur von -78°C bis 100°C während 1 bis 100 h durchgeführt wird, daß die Polymerisierung durch Hydrolyse mittels Ethanol oder Wasser angehalten wird, und daß ein Diblock-Copolymer von Ethylen - (einem) polaren Comonomer/en mittels Filtration oder Extraktion mit Hilfe eines Lösungsmittels gewonnen wird, welches das Homopolymer des entsprechenden polaren Comonomers solubilisiert.

**16.** Anwendung der Dialkylmagnesiumverbindungen mit langen Ketten gemäß der Definition in einem der Ansprüche 10 bis 12 als Makroinitiatoren für die Polymerisierung von polaren Monomeren, die zu Diblock-Copolymeren mit einer Polyethylensequenz führt.

**17.** Anwendung der Dialkylmagnesiumverbindungen mit langen Ketten gemäß der Definition in einem der Ansprüche 10 bis 12 bei der Herstellung von geradkettigen primären Fettalkoholen mittels Oxidation und Hydrolyse.

**18.** Anwendung der Dialkylmagnesiumverbindungen mit langen Ketten gemäß der Definition in einem der Ansprüche 10 bis 12 bei der Herstellung von $\alpha$-Olefinen mittels Thermolyse.

**19.** Anwendung der Dialkylmagnesiumverbindungen mit langen Ketten gemäß der Definition in einem der Ansprüche 10 bis 12 bei der Herstellung von Säuren mittels Umsetzung mit $CO_2$.

**20.** Anwendung der Dialkylmagnesiumverbindungen mit langen Ketten gemäß der Definition in einem der Ansprüche 10 bis 12 zum Fixieren eines Restes vom Typ $R-(CH_2-CH_2)_n-$, wobei R und n der Definition in Anspruch 1 entsprechen, an einem organischen Molekül oder einem Wirkprinzip zum Erhöhen seiner Fettlöslichkeit.